# EUROPEAN PATENT SPECIFICATION

(11) **EP 2 229 967 B1**
(45) Date of publication and mention of the grant of the patent: **15.04.2020**
(21) Application number: 09003798.7
(22) Date of filing: 17.03.2009
(51) Int. Cl.: A61M 5/168

(54) **Cannula assembly and ambulatory infusion system with a pressure sensor made of stacked coplanar layers**
Kanülenanordnung und ambulantes Infusionssystem mit Drucksensor aus koplanaren Schichtstapeln
Ensemble de canule et système de perfusion ambulatoire avec un capteur de pression fabriqué à partir de couches coplanaires superposées

(43) Date of publication of application: 22.09.2010
(73) Proprietor: F. Hoffmann-La Roche AG, 4070 Basel (CH); Roche Diabetes Care GmbH, 68305 Mannheim (DE)
(72) Inventor: Geipel, Andreas, 4665 Oftringen (CH); Kühni, Florian, 3007 Bern (CH); Huwiler, Christoph, 6415 Arth (CH); Haueter, Ulrich, 3506 Grosshöchstetten (CH)
(74) Representative: Rentsch Partner AG

(56) References cited:
- EP-A- 0 009 313
- EP-A- 1 961 436
- EP-B1- 1 207 378
- WO-A-2005/077262
- US-A1- 2008 200 897

## Description

The present invention is directed towards a cannula assembly for ambulatory drug infusion over an extended time period, the cannula assembly comprising a pressure sensor which is made by a stack of coplanar layers. The invention is further directed towards an ambulatory infusion system comprising such a pressure sensor.

Ambulatory infusion systems and devices for the infusion of a liquid drug over an extended time period are known in the art for a number of therapies. In particular, such devices form the basis for a state-of-the-art therapy of Diabetes Mellitus by CSII (Continuous Subcutaneous Insulin Infusion). A corresponding device is disclosed, for example, in the WO2003053498A2 to which reference is made for the general design and features of such devices.

Besides diabetes therapy, those infusion devices may be used for a number of further therapies, such as cancer treatment or pain therapy, without requiring substantial modification. The following description of the present invention mainly refers to CSII therapy without limiting the invention to this specific application.

When using an ambulatory infusion device, a number of error situations or hazardous situations may occur, involving a potential danger for the patient. Occlusions of the infusion cannula or the infusion tubing are especially critical and have to be detected as early as possible. For this purpose, the fluidic pressure of the drug may be continuously monitored. In state-of-the-art devices, this is typically done by measuring the reaction-force exerted by a plunger of the drive system of the infusion device onto a plug of the drug cartridge. Corresponding designs are disclosed, among others, in US6362591. This approach, however, involves a complex and critical electro-mechanical design of the infusion system, substantially adds to the size of the device and involves a number of measuring uncertainties, for example due to friction. It is therefore favorable to directly measure the fluidic pressure of the drug. Pressure measurement arrangements have been designed for this purpose, for example, in WO 2007/093064. Those arrangements, however, involve complex and expensive disposable components. Furthermore, it would be favorable to measure the drug pressure directly at the infusion cannula rather than at the pump outlet.

The patent US 6830558 B2 discloses a flow condition sensor assembly for an infusion device with a first electrode which is secured on a diaphragm and a second electrode which is positioned in a fixed location with respect to the first electrode. An impedance meter is connected to the electrodes. The diaphragm is resilient and may bend into a curved sensor chamber, thus modifying the impedance which may be measured between the first electrode and the second electrode. However, the sensor comprises components of complex structure and is critical in assembly.

Document EP 1 961 436 A1 discloses a cannula assembly having a presure sensor. Document EP 0 009 313 A1 discloses particular pressure sensor designs.

It is the overall objective of the present invention to improve the state of the art by providing devices which allow administration supervision and detection of hazardous situations in a reliable, compact, and cost-efficient way. Therefore, capacitive pressure sensors are provided which may be produced in a cost-efficient and reliable way in a large-scale manufacture and do not require complex machining and assembly steps. A pressure sensor according to the present invention may be arranged directly at an infusion cannula or at the outlet of the infusion pump, upstream of the infusion tubing and the cannula or may be comprised by an ambulatory infusion system at an alternative suited location.

In a first aspect, the invention is directed towards a cannula assembly, the cannula assembly comprising an infusion cannula and a hub, the hub comprising a pressure sensor with a fluid channel, the fluid channel being fluidically arranged upstream of the infusion cannula. The pressure sensor comprises a stack of coplanar layers with:
a) a rigid top layer and a rigid base layer,
b) a resilient metallic electrode layer and a metallic counter-electrode layer, the electrode layer and the counter-electrode layer being electrodes of a sensing capacitor,
c) a spacer layer which has a through cut-out, the through cut-out defining an electrode cavity,
wherein the fluid channel is coupled to the electrode layer such that a fluidic positive pressure of the drug in the fluid channel causes the electrode layer to bend into the electrode cavity, thus modifying the capacitance of the sensing capacitor.

The hub is the extracorporeal portion of the cannula assembly from which the infusion cannula projects. It is typically designed to be attached to the patient's body or skin, for example with a belt or an adhesive layer contacting the skin. The infusion cannula may be directly connected to the fluid channel and be integral with the drug outlet for this type of arrangement. The cannula may be of any design known in the art and may especially be made of metal, such as medical grade stainless steel, or a plastic material, such as Teflon. The hub may be designed such that the cannula is placed into the skin in a substantially perpendicular way or at a smaller angel of, e.g., 10° to 20° to the skin.

In some embodiments, the cannula assembly comprises a fluidic coupler upstream of the pressure sensor and in fluid communication with the fluid channel, the fluidic coupler being configured to couple the cannula assembly to an infusion device. The fludic coupler may be a releasable coupler, such as a Luer coupler or a proprietary coupler. The coupler may alternatively be designed such that the fluidic connection may be established by the patient but may not be released again, for example by providing snapping elements, or the like. Alternatively, the cannula assembly may not comprise a dedicated coupler but may be provided with further components, such as a drug cartridge or a dosing unit readily attached.

The cannula assembly may be designed for direct coupling to an infusion device or for coupling via additional tubing.

The coplanar design of the layers of the pressure sensor allows realizing a flat and sandwich-like structure which is well-suited for large-scale manufacture. The layers forming the stack may be bonded to each other using established technologies such as laminating, adhesive bonding, ultrasonic welding or laser bonding. In advantageous embodiments, the inner layers of the pressure sensor, i.e., the layers which are arranged between the top layer and the bottom layer, in particular the electrode layer, the counter-electrode layer and the spacer layer, are of substantially the same size, thus simplifying the positioning at manufacture. The surface area of the electrode layer and the counter-electrode layer is in particular larger than the area of the through cut-out, such that it is covered by the electrode layer and the counter-electrode layer over its whole area.

The inner layers have a constant thickness over their whole surface area, resulting in their surfaces being coplanar and not showing recesses or the like. This also holds true for the inner surfaces of the top layer and the bottom layer which contact the inner layers. This design allows the single layers to be thin and simplifies the positioning and bonding of the layers. In some embodiments, the outer surfaces of the top layer and the bottom layer, respectively, are coplanar with the surfaces of the inner layers, such that all layers of the stack are coplanar.

While large dimensions and surface areas of the layers are favorable for the manufacture as well as with respect to the capacitance of the sensing capacitor, small dimensions are desired for a compact design. In an infusion system for CSII, the size of the electrode cavity may be in a favorable range of 15mm² to 80mm². The electrode cavity and the fluid cavity may generally have any suited shape according to the overall design requirements. A circular, elliptical or rectangular shape is believed to be especially favorable.

The resulting nominal capacitance of the sensing capacitor is advantageously smaller than 30pF and may especially be in the range of 3pF to 5pF. Other nominal capacities, however, are also possible in dependence of the design constraints. The term 'nominal capacitance' refers to the capacitance of the sensing capacitor in the unloaded state of the pressure sensor, that is, if no pressure is acting electrode layer which would cause the electrode layer to bend. The sensing capacitor is defined by the surface of the electrode layer and the counter-electrode as well as the volume between those layers. Further aspects of the design of the sensing capacitor will become more readily visible as the description proceeds, and in particular from the description of exemplary embodiments.

The fluid channel is advantageously widened to form a fluid cavity of at least the same size and in alignment with the electrode cavity, such that the fluidic pressure of the drug in the fluid channel equally acts on the whole surface electrode layer in the area which may bent under the influence of a fluidic drug pressure, i.e., the are which covers the through cut-out in the spacer layer. The fluid channel may be formed into the base layer for example during injection-molding of the base layer or may be formed by punching, milling, or another technology known in the art. Alternatively, a further foil layer may be provided with embedded fluid channels and/or cavities. Those channels and cavities may be formed by hot embossing or deep drawing.

The fluid channel is advantageously arranged inside the coplanar stack of layers which is bordered by the top layer and the base layer, respectively. The fluid channel is further advantageously separated from the electrode layer by an electrode sealing layer which is attached to the electrode layer to form a combined sandwich layer. The electrode sealing layer is resilient in order to allow it to bend together with the electrode layer.

The pressure which causes the electrode layer to bend is the differential pressure between the drug pressure in the fluid channel and the reference pressure in the electrode cavity. A fluidic drug pressure that is larger than the reference pressure is referred to as 'positive pressure' and a fluidic drug pressure that is smaller than the reference pressure is referred to as 'negative pressure'. If the electrode cavity is vented to the environment, the electrode cavity is filled with air and the reference pressure is the environmental pressure. Alternatively, the electrode cavity may be hermetically sealed and filled with air or another fluid, typically a gas, during manufacture. In this case, the reference pressure is the constant pressure of this gas.

In some embodiments, foils are used at least for the electrode layer and the spacer layer. The electrode layer is a metal foil or a compound of a metal foil and a non-metal carrier foil or is made by metalizing a non-metal carrier foil, for example by chemical metallization, galvanic metallization or lacquering.

Foils are advantageously with respect to their inherent elastic and/or resilient properties and generally good processing properties in manufacture. For manufacture, the foils may be supplied on wheels or sheets which allow fast parallel and/or serial processing. Different mechanical properties, such as bending rigidities, may be achieved by appropriate selection of the foil materials and thickness.

Metalized foils are advantageously substantially unstructured and are covered with metal over their whole surface, thus avoiding critical structuring steps, such as partial etching, partial metallization, or the like. Therefore, the large-scale manufacture may be carried out in an economic and cost-efficient way. The sensors may be assembled by simply stacking and attaching the foil layers as supplied form wheels or as sheets to each other. Subsequently, the foil sandwich may be cut or punched as known in the art in order to separate individual sensors.

Providing the spacer layer as foil is favorable since it allows especially simple manufacture of the through cut-out by cutting or punching. Those steps may be performed simultaneous for a large number of sensors in one step, prior to bonding the foil with other foils and prior to separating the individual sensors as described above. As compared to the electrode layer, the spacer layer may be more rigid to be substantially stiff during operation and/or handling.

The further layers of the stack may be made from foils, too. Alternatively, some layers, in particular the top layer and the base layer, are made by plastic parts different from foils such as injected molded parts. For such an arrangement, the top-layer and/or the base layer may be integral with the hub housing and form a supporting structure or frame inside which the other layers are arranged. The counter-electrode layer may be made by a metal foil or metalized foil like the electrode layer, or may be made by metallization of the top layer.

For the layers made of foils, a variety of known materials may be used, for example polymeric materials such as PMMA, PC, PSU, or PEEK. A typical advantageous range for the foil thickness is from 50µm to 200µm.

In some embodiments, the spacer layer is arranged between and in contact with the electrode layer and the counter-electrode layer. For this type of embodiment, the coplanar cap surfaces of the electrode cavity are given by the electrode layer and the counter-electrode layer, respectively, while the shell surface of the electrode cavity is the inner flange of the cut-out.

In some of those embodiments, the electrode layer, the counter-electrode layer and the electrode cavity are designed such that, upon a maximum threshold pressure, the electrode layer touches the counter-electrode layer. The major influence factors that may be adjusted for that purpose are the shape and dimensions of the electrode cavity as well as the bending rigidity of the electrode layer and the electrode sealing layer. Under the given dimensional restrictions, those parameters may be adjusted for a given maximum threshold pressure by routine computational and/or experimental work.

The design may be such that the electrode layer and the counter-electrode layer establish a galvanic contact upon the electrode layer touching the counter-electrode layer at the maximum threshold pressure. This galvanic contact may be detected in order to trigger the immediate generation of an occlusion or overpressure alarm.

Alternatively, at least one of the electrode layer and the counter-electrode layer comprises an insulation layer which faces the other of the electrode layer and the counter-electrode layer, respectively, thus preventing a galvanic contact to be established by the electrode layer and the counter-electrode layer. In this case, the capacitance of the sensing capacitor is saturated when the electrode layer and the counter-electrode layer touch each other at the maximum threshold pressure.

The fluid channel of the pressure sensor may comprise a flow restrictor. Such a flow restrictor may be arranged downstream of the bending area. If drug is administered, the flow restrictor increases the fluidic pressure upstream of the flow resistor according to the law of Hagen-Poiseuille. Due to the increased pressure, the bending of the electrode layer and, thus, the change in capacitance are increased. The flow restrictor may be realized by increasing the length of the fluid path downstream of the fluid cavity and/or by reducing the hydraulic diameter downstream of the fluid cavity. Therefore, the fluid channel may have a meander- shaped portion.

The pressure sensor may comprise a reference capacitor in addition to the sensing capacitor. The reference capacitor comprises a reference electrode layer and a reference counter-electrode layer. Providing a reference capacitor which is vented to the environment is advantageous since it allows compensating the signal provided by the sensing capacitor for undesired environmental effects such as changing temperature and atmospheric pressure. The reference capacitor is advantageously of the same design as the sensing capacitor. It advantageously further has the same or a similar nominal capacitance as the sensing capacitor and comprises a reference pressure cavity which is vented to the environment via a reference venting aperture such that the environmental pressure acts on and may bend the reference electrode layer. Any change in the environmental conditions will accordingly act on the sensing capacitor and the reference capacitor in the same way.

In some embodiments comprising a reference capacitor, the sensing capacitor and the reference capacitor are arranged next to each other in the same plane. Alternatively, the sensing capacitor and the reference capacitor are arranged in a stacked way, thus reducing the surface area of the stack while increasing its thickness.

In some embodiments comprising a reference capacitor, either of the electrode layer and the reference electrode layer or the counter-electrode layer and the reference counter-electrode layer are a common layer, thus forming a common electrode.

In some embodiments, only the electrode layer is designed to bend under the influence of the fluidic pressure of the drug, while the counter-electrode layer maintains its planar shape. Alternatively, the fluid channel is coupled to the counter-electrode layer such that a fluidic pressure of the drug in the fluid channel results in the counter-electrode layer being bent, too, thus modifying the capacitance of the sensing capacitor. This measure increases the capacitance variation resulting from a fluidic pressure variation, and, thus, the sensitivity of the pressure sensor.

The electrode layer, the counter-electrode layer and the fluid channel may be arranged such that the capacitance of the sensing capacitor increases upon increasing fluidic pressure of the drug in the fluid channel. Alternatively, the arrangement may be such that the capacitance of the sensing capacitor decreases upon increasing fluidic pressure of the drug in the fluid channel. Further details on both kinds of embodiments are given below in the context of exemplary embodiments.

In some embodiments, the pressure sensor comprises a further sensing capacitor, the further sensing capacitor comprising a further electrode layer and a further counter-electrode layer, the further electrode layer, the further counter-electrode layer and the fluid channel being arranged such that the pressure in the fluid channel modifies the capacitance of the sensing capacitor and the capacitance of the further sensing capacitor in opposed ways.

The sensing capacitor and the further sensor capacitor of such an embodiment are advantageously designed in an analogue way. Both capacitors especially comprise a stack of coplanar layers, wherein some of the layers may be common for the pressure sensor and the further pressure sensor. This kind of embodiment is especially favorable with respect to sensitivity as well as the compensation of environmental effects, since the fluidic drug pressure in the flow channel modifies the capacitances of the sensing capacitor and the further sensing capacitor in opposite ways, while environmental effects act on both capacitors in the same way. The geometric arrangement of sensing capacitor and the further sensing capacitor may be next to each other or may be stacked.

According to a further aspect, the invention is directed towards an ambulatory infusion system for the infusion of a liquid drug into a patient's body over an extended time period, the ambulatory infusion system comprising:
a) a drug container,
b) an infusion cannula which is fluidically coupled to the drug container and is configured to be placed into the patient's subcutaneous tissue for drug infusion,
c) an infusion device, the infusion device being coupled to the drug container for controlled drug administration via the infusion cannula,
c) a pressure sensor with a fluid channel, the fluid channel being fluidically arranged between the drug container and the infusion cannula, the pressure sensor defining a sensing capacitor,
d) a measuring unit which is coupled to the pressure sensor, the measuring unit being configured to evaluate a variation of the capacitance of the sensing capacitor.

The pressure sensor comprises a stack of coplanar layers with:
e) a rigid top layer and a rigid base layer,
f) a resilient metallic electrode layer and a metallic counter-electrode layer, the electrode layer and the counter-electrode layer being electrodes of the sensing capacitor,
g) a spacer layer which has a through cut-out, the through cut-out defining an electrode cavity,
wherein the fluid channel is coupled to the electrode layer such that a positive fluidic pressure of the drug in the fluid channel causes the electrode layer to bend into the electrode cavity, thus modifying the capacitance of the sensing capacitor.

The overall architecture of the infusion device may follow a design as known in the art and comprise the elements and technical features typical for such devices, as disclosed, for example, in WO2003/053498A2. The infusion device may especially be a computer-controlled syringe-driver pump but may alternatively be of a different type and comprise, for example, a micro membrane pump or a dosing unit as disclosed in EP1970677A1.

The measuring unit comprises a capacitance measurement circuit. For this purpose, it may comprise elements known in the art, such as filters, amplifiers, reference voltage and/or reference current supplies, analogue-to-digital converters, and the like. The capacitance measurement circuit may be based on a capacitance-to-voltage converter such as the integrated circuit CAV424 as available from Analog Microelectronics GmbH, Germany. If the pressure sensor comprises a sensing capacitor and a further sensing capacitor, the measuring circuit is advantageously designed to measure the capacitances of both the sensing capacitor and the further sensing capacitor. The measuring unit may further detect the presence of a galvanic contact between the electrode and the counter-electrode,

The pressure sensor may be comprised by a main housing of the infusion device. This is the case if the whole system is disposable. Alternatively, it may be comprised by a disposable, such as the drug container. In a further alternative, the pressure sensor is included in an adapter which is coupled to the drug container and is coupled to an infusion tubing. In a further alternative, the drug sensor is comprised by a cannula assembly as described above.

The measuring unit and/or a controller of the infusion device are configured to generate a warning or an alarm in case the fluidic pressure or a variation of the fluidic pressure indicates the occurrence of an error condition, such as an occlusion. Corresponding circuit designs and algorithms are known in the art.

Whereas the position of the pressure sensor may differ from the position of the pressure sensor as disclosed above in the context of a cannula assembly, all embodiments of the pressure sensor as described above may also be embodiments of a pressure sensor in an ambulatory infusion system according to this aspect of the invention.

It should be noted that a pressure sensor comprising a stack of coplanar layer as described above and as described below in the framework of exemplary embodiments also defines an invention on its own which the applicant reserves the right to pursue separately from this application.

In the following, exemplary embodiments of the present invention are described in more detail with reference to the figures.
Figure 1 schematically shows the overall architecture of an ambulatory infusion system according to the present invention.
Figure 2 exemplary shows a drug volume curve and a corresponding pressure curve as it may be measured in an infusion system according to the present invention.
Figure 3 and Figure 4 schematically show exemplary cannula assemblies according to the present invention.
Figure 5, Figure 6, and Figure 7 show stacks of coplanar layers of exemplary pressure sensors in accordance with the present invention.
Figure 8a shows a stack of coplanar layers of a further exemplary pressure sensor according to the present invention and Figure 8b shows a corresponding bottom view of the sensor.
Figure 9 and Figure 10 show stacks of coplanar layers of further exemplary pressure sensors according to the present invention.
Figure 11 schematically shows a pressure sensor in accordance with the present invention.
Figure 12a shows a stack of coplanar layers of a further exemplary pressure sensor according to the present invention and Figure 12b shows a corresponding bottom view of the sensor.

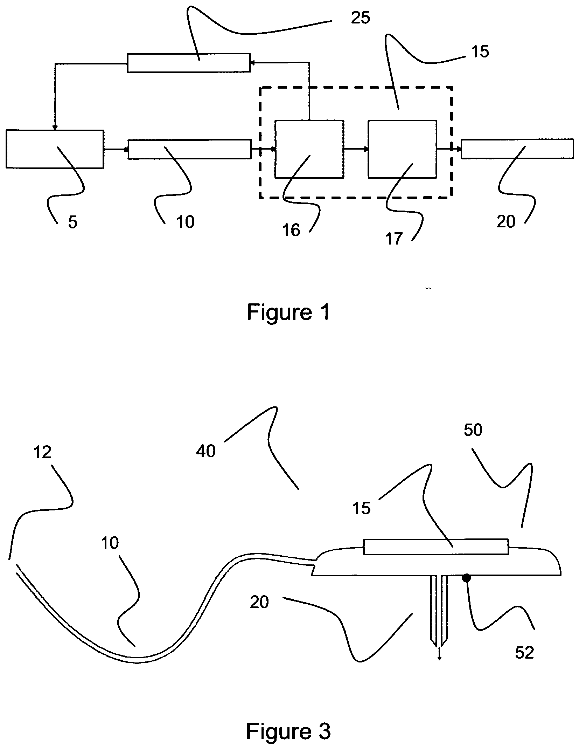

Figure 1 schematically shows the overall architecture of an ambulatory infusion system according to the present invention. The infusion system comprises an infusion device 5, an infusion tubing 10, a pressure sensor 15 with a sensing capacitor 16 and an optional flow restrictor 17, and an infusion cannula 20 which is designed to be placed into the patient's subcutaneous tissue for the drug infusion.

The infusion system further comprises a measuring unit 25 which operatively couples the pressure sensor 15 to a controller of the infusion device 5. While shown as separate unit, the measuring unit 25 may be, fully or in part, integral with the infusion device 5. The measuring unit 25 is configured to process and evaluate an electrical signal generated by the pressure sensor 15. The measuring unit 25 is especially configured to detect the presence of a substantially continuous pressure increase over time as resulting from an occlusion of the fluidic system, for example of the infusion cannula 20. The measuring unit 25 may further or alternatively be configured to supervise the drug administration by the infusion device 5 by detecting the presence of short pressure pulses by which the administration of each drug pulse is accompanied. The infusion device 5 may be designed for basal drug administration by administering small drug pulses every few minutes, as it is the case, for example, for typical syringe-driver pumps. Each of these drug pulses is associated with a pressure peak which may be detected by the pressure sensor 15 and the presence of which may be determined by the measuring unit 25. The substantially continuous administration of larger drug amounts, such as the administration of drug boli, is associated with a substantially constant pressure plateau during the administration which also may be determined by the measuring unit 25.

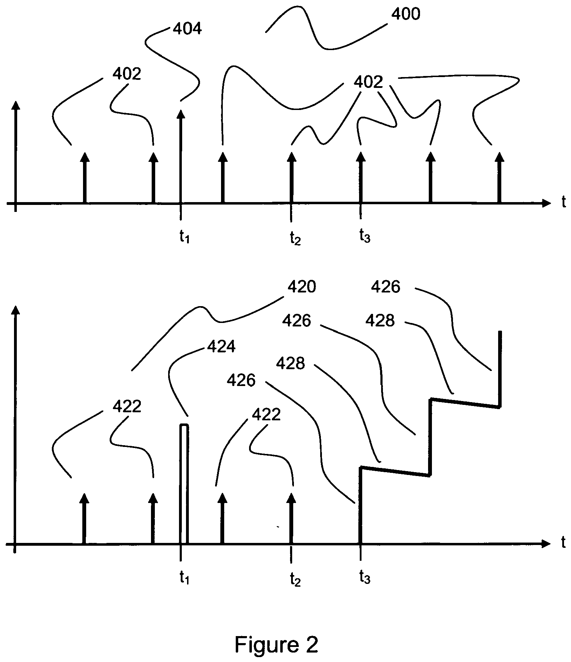

Figure 2 schematically shows an exemplary administration volume curve 400 and a corresponding pressure curve 420 as it may measured by the pressure sensor 15 as function of time t. The infusion system administers small basal drug pulses 402 in equidistant time intervals, resulting in a quasi continuous basal administration. Each basal drug pulse 402 is reflected by a corresponding basal pressure pulse 422. At time t₁, and additional on-demand bolus 404 of with a larger volume is administered. The corresponding temporary bolus pressure increase 424 may be a short pulse or may have a plateau as shown in the example of Figure 2, dependent on the administration regime and the time required for the administration. At some point in time between t₂ and t₃, an occlusion is established, that is, the fluid path is blocked. Subsequent basal administrations results in a stepwise pressure increase, with a step 426 occurring each time a basal drug pulse 402 is administered. Between the steps 426, the pressure may stay substantially constant or slightly decrease due to the elasticity of the fluidic system. The administration of a drug bolus would result in an even steeper pressure increase (not shown).

Figure 3 schematically shows an exemplary cannula assembly 40 according to the present invention. The pressure sensor 15 is integrated into the extra-corporal hub 50 which is advantageously made from plastics. This hub further comprises a fluidic coupler (not shown) for coupling the infusion tubing 10 to the cannula assembly and an electric coupler (not shown) for coupling the pressure sensor 15 to the infusion device 5. At the device-side proximal end 12 of the infusion tubing 10 a fluidic connector (not shown) such as a Luer connector or the like is provided for coupling to the infusion device. The infusion cannula 20 projects out of the hub 50 and may be made of a soft material, such as Teflon, or metal, such as medical grade stainless steel and is designed as known in the art. The hub 50 is attached to the patient's skin with its skin-contacting surface 52 via a skin compatible adhesive layer. The pressure sensor 15 may be designed in accordance with any embodiment of the present invention.

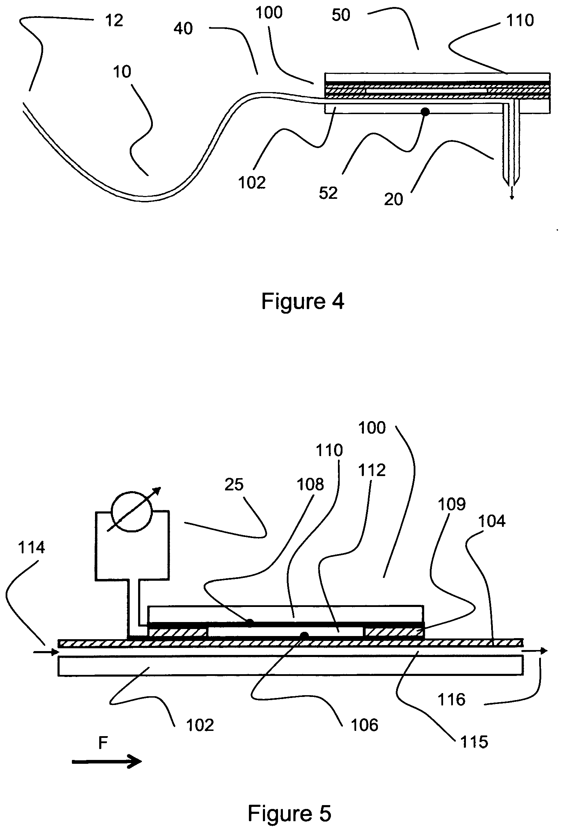

Figure 4 schematically shows an alternative design of a cannula assembly 40 according to the present invention. The pressure sensor 15 is realized as a stack 100 of coplanar layers. The base layer 102 and the top layer 110 are integral with the rigid structure of the hub 50. Exemplary designs of the pressure sensor 15 and in particular of the stack 99 are described in detail in the following.

The measuring unit 25 as shown in Figure 1 may be integrated into the infusion pump 5 or may be, fully or in part, be integrated into the hub 50 of the cannula assembly 40 and in close proximity to the pressure sensor 15. In particular, the capacitance measurement circuitry is advantageously located close to the pressure sensor 15 in order to minimize signal distortions of the low-energy measurement signal and in order to minimize or fully avoid undesired wiring capacities in parallel to the sensing capacitor. Further components of the measuring unit 25, such as an analogue-to-digital converter for the measurement signal may be provided in the hub 50. In some designs, all components of the measuring unit 25, including the circuitry for evaluating the signals generated by the pressure sensor 15, are located in the hub 50.

Those components of the measuring unit 25 which are comprised by the hub 50 are designed for single-use and are disposed as part of the cannula assembly after some days of use. The measuring unit 25 may alternatively, fully or in part, be comprised by a small separate housing which is configured to be removable attached to the hub 50. This kind of design allows re-usage of the measuring unit 25 while still allowing it to be located in proximity to the pressure sensor 15.

Figure 5 schematically shows a stack 100 of coplanar layers of an exemplary pressure sensor 15 in a cross-sectional view. Like in the following figures, the dimensions are chosen for best clarity and visibility of the design and do not necessarily correspond to the actual dimensions. The layers of the stack 100 are arranged in a sandwich-like way. The stack 100 comprises a substantially rigid base layers 102 and top layers 110, respectively, between which the other layers are arranged. The electrode layer 106 and the counter-electrode layer 108 of the sensing capacitor are separated by an electrode spacer layer 109 which defines the distance between the electrode and the counter-electrode. A fluid channel 115 for conducting the drug from a drug inlet 114 to the drug outlet 116 is limited by the base layer 102 and an electrode sealing layer 104, the electrode sealing layer 104 separating the drug from the electrode layer 106. The electrode spacer layer 109 comprises a through cut-out which forms the electrode cavity 112. The through cut-out and, thus, the top view of the electrode cavity 112 are advantageously circular elliptical or substantially rectangular. The combination of the electrode layer 106 and the electrode sealing layer 104 is resilient such that they can bend together perpendicular to the flow direction F and into the electrode cavity 112. The fluid channel 115 is widened to a fluid cavity (not visible) of the same size and in alignment with the electrode cavity

The electrode cavity 112 may be completely encapsulated such that the fluidic drug pressure is measured with respect to a constant reference pressure inside the electrode cavity 112. Alternatively, the electrode cavity 112 may be vented to the environment by a venting aperture (not shown), such that 'positive pressure' and 'negative pressure' are defined with respect to the environmental air pressure at any point in time.

The electrode spacer layer 109 is made from a foil which is connected with the electrode layer 106 and the counter-electrode layer 108 via bonding or laminating. The base layer 102 and the top layer 110 are made from rigid plastics and may be part of the hub housing.

In combination, the electrode layer 106 and the counter-electrode layer 108 form the electrodes of a sensing capacitor the dielectric of which is largely given by the air or gas inside the electrode cavity 112 with the electrode distance being defined by the thickness of the electrode spacer layer 109. In addition, there is a parallel offset capacitor. The offset capacitor is given by the surface of the electrode layer 106 and the counter-electrode layer 108 outside the electrode cavity 112 with the electrode spacer layer 109 serving as dielectric. A further component of the offset capacitor is given by the wiring. In order to minimize the undesired capacitance offset of the sensing capacitor, the surface of the electrode layer 106 and the counter-electrode layer 108 outside the electrode cavity 112 should be kept as small as possible and the wiring for electrically connecting the electrode layer 106 and the counter-electrode layer 108 should be as short as possible.

In the area of the electrode cavity 112, the fluid channel is widened to form a fluid cavity such that the fluidic pressure of the drug inside the fluid channel 115 acts on the whole surface area of the electrode layer 106 in the bending area, that is, the area of the electrode cavity 112.

For a positive pressure of the drug in the fluid channel 115, the electrode sealing layer 104 and the electrode layer 106 will bend into the sensing electrode cavity 112, thus reducing the distance between the electrode 106 and the counter-electrode 108 and accordingly increasing the capacitance of the sensing capacitor. In an analogue way, a negative pressure of the drug in the fluid channel 115 results the electrode sealing layer 104 and the electrode layer 106 to bend into the fluid channel 115, thus increasing the distance between the electrode layer 106 and the counter-electrode 108 and reducing the capacitance of the sensing capacitor. The electrode layer and the counter-electrode layer are electrically coupled to the measuring unit 25 which is schematically represented by an impedance meter.

The bending rigidity of the electrode layer 106 and the electrode sealing layer 104, as well the thickness of the electrode spacer layer 109 may be chosen such that the electrode layer and the counter-electrode layer do not come into contact at the highest fluidic pressure in the fluid channel 115 which may occur, for example, in case of an occlusion. In this case, the capacitance measurement circuit is designed for capacitance measurement only. However, the design may alternatively be such that, upon a given threshold maximum pressure, the electrode layer 106 touches the counter-electrode layer 108, thus establishing a galvanic contact. This galvanic contact may additionally be detected by the capacitance measurement circuit of the measuring unit 25 or by a controller of the infusion device.

While several modifications and variations are disclosed and discussed in further exemplary embodiments below, the principle design is the same and follows the description as given with reference to Figure 5.

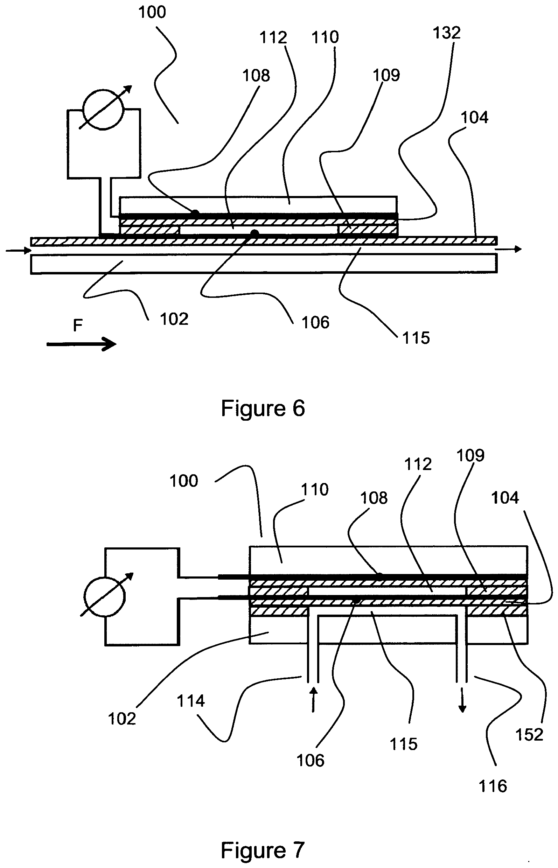

Figure 6 shows a stack 100 of coplanar layers of a further exemplary pressure sensor 15. Like in the following figures, the same reference signs are used for elements having substantially the same function. For clarity reasons, some of those elements are not referenced which are not relevant for the specific embodiments.

In the exemplary embodiment shown in Figure 6, the counter-electrode layer 108 comprises an insulation layer which faces the electrode layer 106. The insulation layer 132 results in the electrode layer 106 and the counter-electrode layer 108 not establishing a galvanic contact when touching each other. Instead, the capacitance of the sensing capacitor shows saturation in this case. Additionally or alternatively to the insulation layer 132, an insulation layer may be comprised by the electrode layer 106 in an analogue way.

Figure 7 shows a stack 100 of coplanar layers of a further exemplary pressure sensor 15. For this design the drug inlet 114 and the drug outlet 116 are perpendicular to the flow direction F of the drug inside the sensor. For the drug inlet 114 and the drug outlet 116, bores (not referenced) are provided in the base layer 102. A fluid channel spacer layer 152 is provided between the base layer 102 and the electrode sealing layer 104. The base layer 102, the fluid channel spacer layer 152, and the electrode sealing layer 104, in combination, define the fluid channel 115.

Instead of providing the dedicated fluid channel spacer layer 152, the fluid channel 115 may be formed by structuring the base layer 102 as described above.

In a further exemplary design (not shown), one of the drug inlet 114 and the drug outlet 116 is tangential with the stacked layers and the flow direction F, while the other of the drug inlet 114 and the drug outlet 116 is perpendicular to the stacked layers. This is the case for the design of the stacked layers 100 as shown in Figure 4. The drug outlet 116 may especially be perpendicular to the stacked layers for cannula assembly designs where the cannula is placed into the patient's subcutaneous tissue substantially perpendicular to the skin. In contrast, a design with the drug outlet 116 being tangential with the stacked layers may be advantageous for a design of the cannula assembly where the infusion cannula is placed into the patients skin with a small angle of, for example, 10° to 20°.

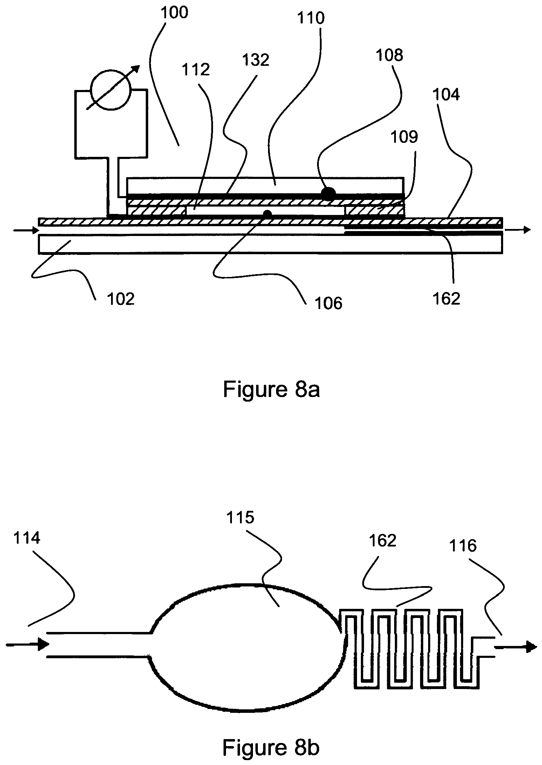

Figure 8a shows a further stack 100 of coplanar layers of an exemplary pressure sensor 15. The pressure sensor is designed similar to the pressure sensor shown in Figure 5 and described above, but comprises an additional flow restrictor 162 which serves as additional fluidic resistance downstream of the sensing capacitor, thus increasing the pressure increase that can be measured by the pressure sensor during drug administration.

Figure 8b schematically shows the fluidic arrangement according to Figure 8a in a top-view or bottom view. The fluid channel 115 is widened to form an elliptical fluid cavity in the area of the sensing capacitor, corresponding to the shape of the electrode cavity 112. The flow-restrictor 162 is arranged downstream of the bending-area and is meander-shaped. This shape is favorable for the flow restrictor 162 since it allows realizing a long fluid path with a short distance in the flow direction F, thus enabling a compact design. However, other alternative shapes are known in the art and may be used as well.

The fluid channel 15, including the meander-shaped flow-restrictor 162, may be realized by structuring the base layer 102. Alternatively, a further layer (not visible), such as a foil layer, may be provided between the base layer 102 and the electrode sealing layer 106. This additional layer has a meander-shaped through cut-out or punching which forms the side walls of the fluid channel 115 in the area of the flow restrictor 162.

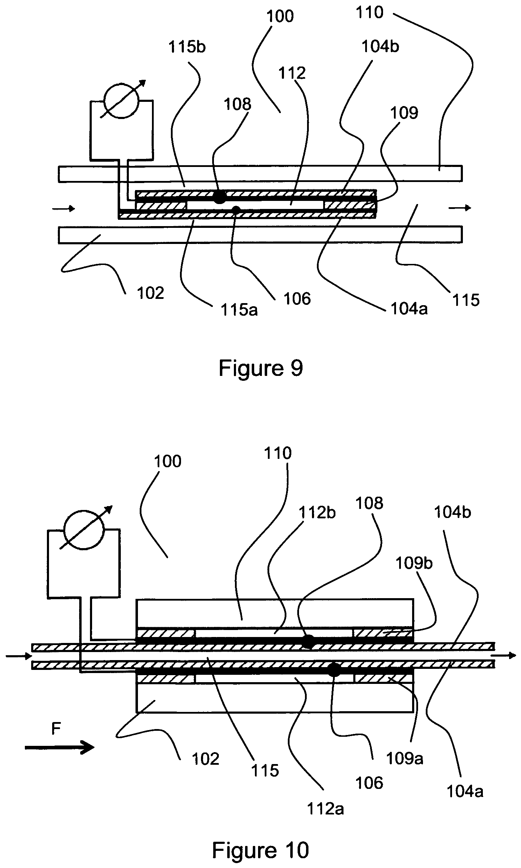

Figure 9 shows a stack 100 of coplanar layers of a further exemplary pressure sensor 15. The design is similar to the arrangement shown in Figure 4 and described above. However, the sensing capacitor is arranged such that the fluidic pressure in the fluid channel 115 acts on both the electrode layer 106 and the counter-electrode layer 108. For this arrangement, both the electrode layer 106 and the counter-electrode layer 108 are allowed to bend under the influence of the fluidic pressure, wherein a positive pressure results in the electrode layer 106 and the counter-electrode layer 108 bending towards each other. Enabling both the electrode layer 106 and the counter-electrode layer 108 to bend under the influence of the fluidic pressure significantly increases the sensitivity of the pressure sensor.

For separating the drug in the fluid channel 115 from the counter-electrode layer 108, a counter-electrode sealing layer 104b is provided in this arrangement. While not visible in Figure 8, the base layer 102 and the electrode sealing layer 104a as well as the top layer 110 and the counter-electrode sealing layer 104b are spaced by fluid channel spacer layers, such that the fluid channel 115 is divided into an electrode fluid channel 115a between the base layer 102 and the electrode sealing layer 104a, and into a counter-electrode fluid channel 115b between the top layer 110 and the counter-electrode-sealing layer 104b. Alternatively, the base layer 102 and the top layer 110 are part of a solid structure, e.g., a housing structure of the hub which supports the inner layers.

The fluidic pressure in the electrode fluid channel 115a acts on the electrode layer 106 and the fluidic pressure in the counter-electrode fluid channel 115b acts on the counter-electrode 108 in the bending area. The stack 100 may optionally comprise an insulation layer as discussed above with reference to Figure 6.

Figure 10 shows a stack 100 of coplanar layers of a further exemplary pressure sensor 15 which allows bending of both the electrode 106 as well as the counter-electrode 108. Here, the fluid channel 115 is arranged between the electrode 106 and the counter-electrode 108. For this arrangement, a positive pressure of the drug in the fluid channel 115 results in the electrode layer 106 and the counter-electrode layer 108 bending away from each other, thus increasing the electrode distance. The electrode layer 106 is separated from the base layer 102 by the electrode spacer layer 109a and the counter-electrode layer 108 is separated from the top layer 110 by the counter-electrode electrode spacer layer 109b. The electrode spacer layer 109a comprises an electrode cavity 112a into which the electrode layer 106 may bend and the counter-electrode electrode spacer layer 109b comprises a counter-electrode cavity 112b into which the counter-electrode layer 108 may bend under the influence of a positive fluidic pressure in the fluid channel 115.

Since for this type of embodiment the fluid channel 115 is arranged between the electrode layer 106 and the counter-electrode layer 108, respectively, the dielectric properties of the electrode sealing layer 104a and the counter-electrode sealing layer 104b, respectively, as well as the dielectric properties of the drug in the fluid channel 115 influence the capacitance of the sensing capacitor.

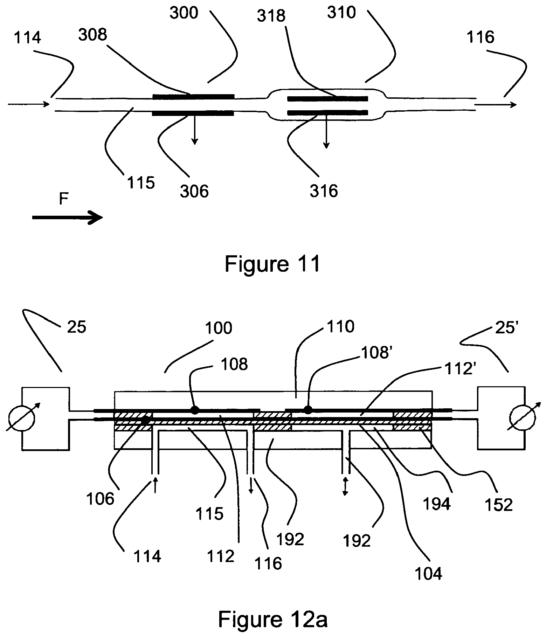

Figure 11 schematically shows an arrangement of a further exemplary pressure sensor according to the present invention. The pressure sensor comprises a sensing capacitor 300. It additionally comprises a further sensing capacitor 310. The sensing capacitor 300 and the further sensing capacitor 310 are fluidically arranged in series in the fluid channel between the drug inlet 114 and the drug outlet 116. The sensing capacitor 300 comprises an electrode layer 306 and a counter-electrode layer 308. The further sensing capacitor 310 comprises a further electrode layer 316 and a further counter-electrode layer 318. The further sensing capacitor 310 is designed such that a positive fluidic pressure of the drug in the fluid channel 115 results in the further electrode layer 316 and the further counter-electrode layer 318 bending towards each other, thus decreasing the corresponding electrode distance and increasing the capacitance of the sensing capacitor. The sensing capacitor 300 is designed such that a positive fluidic pressure of the drug in the fluid channel 115 results in the electrode layer 306 and the counter-electrode layer 308 bending away from each other, thus increasing the corresponding electrode distance and increasing the capacitance of the further sensing capacitor. The sensing capacitor 300 may be designed according to Figure 10 while the further sensing capacitor 310 may be designed according to Figure 9. It will be appreciated by a person skilled in the art that the sensing capacitor 300 and the further sensing capacitor 310 are interchangeable. While the sensing capacitor 300 and the further sensing capacitor 310 are shown behind each other with respect to the flow direction F, they may alternatively be arranged in a stacked way for compactness reasons. Alternatively to being arranged in series, the sensing capacitor 300 and the further sensing capacitor 310 may be in a parallel fluidic arrangement.

Figure 12a shows a stack 100 of coplanar layers of a further exemplary pressure sensor 15 according to the present invention. The sensing capacitor of this embodiment is designed according to Figure 5 with the drug inlet 114 and the drug outlet 116 being arranged according to Figure 7. In addition, the stack 100 implements a reference capacitor.

For this purpose, the stack is enlarged such that the electrode layer 106 covers a reference pressure cavity 194 which is vented to the environment via a reference venting aperture 192. The reference pressure cavity 194 fulfills an analogue function to the fluid cavity of the fluid channel 115 for the sensing capacitor. A reference electrode cavity 112' as well as a reference counter-electrode layer 108' are arranged in an analogue way to the electrode cavity 112 and the counter-electrode layer 108, respectively. The two capacitors are separated by a separation area 196 in which the sandwich of the electrode layer 106 and the electrode sealing layer 104 is fixed to the fluid channel spacer layer 152. Accordingly, the electrode layer 106 may bend into the sensing cavity 112 under the influence of the fluidic pressure of the drug in the fluid channel 115 and may independently bend into the reference electrode cavity 112' under the influence of a positive air pressure in the reference pressure cavity 194. Similarly, the electrode layer 106 may bend into the fluid channel 115 and may independently bend into the reference pressure cavity 194 under the influence of a negative pressure in the fluid channel 115 or the reference pressure cavity 194, respectively.

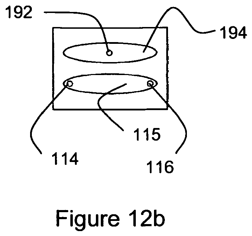

Figure 12b schematically shows the fluidic arrangement according Figure 12a in a bottom-view. The fluidic channel 115 is widened to an elliptic fluid cavity in the area of the sensing capacitor with the drug inlet 114 and the drug outlet 116 being arranged at opposed ends of the long principal axis of the ellipse. The reference pressure cavity 194 is of the same design and size with the central reference venting aperture 192 being arranged in the center of the ellipse. The reference venting aperture 192 may be sealed with a membrane (not shown) which is air permeable but prevents dust and humidity from entering the reference pressure cavity 194. Due to the symmetric design of the sensing capacitor and the reference capacitor, the nominal capacitances of both capacitors are identical besides tolerances. However, the nominal capacitances may alternatively be different by design.

Alternatively to the side-by-side design shown in Figure 12a and Figure 12b, respectively, the sensing capacitor and the reference capacitor may be arranged in a stacked way with one of the capacitors being arranged on top of the other capacitor.

The capacitance of the reference capacitor is measured by a reference capacitance measuring unit 25' which may be integral with the measuring unit 25.

### Reference signs

- 5: infusion pump
- 10: infusion tubing
- 12: proximal tubing end
- 15: pressure sensor
- 16: sensing capacitor
- 17: flow restrictor
- 20: infusion cannula
- 25: measuring unit
- 25': reference capacitance measuring unit
- 40: cannula assembly
- 50: hub
- 52: skin contacting surface
- 100: stack of coplanar layers
- 102: base layer
- 104, 104a, 104b: sealing layer
- 106: electrode layer
- 108: counter-electrode layer, reference counter electrode layer further counter-electrode layer
- 108': reference counter-electrode layer
- 109, 109a, 109b: spacer layer
- 110: top layer
- 112, 112', 112a, 112b: cavity
- 114: drug inlet
- 115, 115a, 115b: fluid channel
- 116: drug outlet
- 132: insulation layer
- 152: fluid channel spacer layer
- 162: flow restrictor
- 192: reference venting aperture
- 194: reference pressure cavity
- 196: separation area
- 300: sensing capacitor
- 306: electrode layer
- 308: counter-electrode layer
- 310: further sensing capacitor
- 316: further electrode layer
- 318: further counter-electrode layer
- 400: administration volume curve
- 402: basal drug pulse
- 404: bolus pulse
- 420: pressure curve
- 422: basal pressure pulse
- 424: bolus pressure increase
- 426: occlusion pressure step
- 428: occlusion pressure plateau

## Claims

1. Cannula assembly (40), comprising an infusion cannula (20) and a hub (50), the hub (50) comprising a pressure sensor (15) with a fluid channel (115), the fluid channel (115) being fluidically arranged upstream of the infusion cannula (20), **c**
**haracterized by** the pressure sensor (15) comprising a stack (100) of coplanar layers, the stack of coplanar layers comprising:
a) a rigid top layer (110) and a rigid base layer (102),
b) a resilient metallic electrode layer (106) and a metallic counter-electrode layer (108), the electrode layer (106) and the counter-electrode layer (108) being electrodes of a sensing capacitor,
c) a spacer layer (109) which has a through cut-out, the through cut-out defining an electrode cavity (112),
wherein the fluid channel (115) is coupled to the electrode layer (106) such that a positive fluidic pressure of the drug in the fluid channel (115) causes the electrode layer (106) to bend into the electrode cavity (112), thus modifying the capacitance of the sensing capacitor, and
**wherein** the electrode layer (106) and the spacer layer (109) are made from foils and the layers which are arranged between the top layer (110) and the bottom layer (102) are of substantially the same size.

2. Cannula assembly (40) according to Claim 1, **characterized by** a fluidic coupler upstream of the pressure sensor (15) and in fluid communication with the fluid channel (115), the fluidic coupler being configured to couple the cannula assembly to an infusion device.

3. Cannula assembly (40) according either of the preceding claims, **characterized in that** the spacer layer (109) is arranged between and in contact with the electrode layer (106) and the counter-electrode layer (108).

4. Cannula assembly (40) according to Claim 3, **characterized in that** the electrode layer (106), the counter-electrode layer (108) and the electrode cavity (112) are designed such that, upon a maximum threshold pressure, the electrode layer (106) touches the counter-electrode layer (108).

5. Cannula assembly (40) according to Claim 4, **characterized by** the electrode layer (106) and the counter-electrode layer (108) establishing a galvanic contact upon the electrode layer (106) touching the counter-electrode layer (108).

6. Cannula assembly (40) according to Claim 4, **characterized by** at least one of the electrode layer (106) and the counter-electrode layer (108) comprising an insulation layer (132) facing the other of the electrode layer (106) and the counter-electrode layer (108), respectively, thus preventing a galvanic contact to be established by the electrode layer (106) and the counter-electrode layer (108).

7. Cannula assembly (40) according to either of the preceding claims, **characterized by** a flow restrictor (17, 162) in the fluid channel (115).

8. Cannula assembly (40) according to either of the preceding claims, **characterized by** a reference capacitor comprising a reference electrode layer (106) and a reference counter-electrode layer (108').

9. Cannula assembly (40) according to Claim 8, **characterized by** either of the electrode layer (106) and the reference electrode layer (106) or the counter-electrode layer (108) and the reference counter-electrode layer (108') being a common layer.

10. Cannula assembly (40) according to either of the preceding claims, **characterized by** the fluid channel (115a, 115b) being coupled to the counter-electrode layer (108) such that a fluidic pressure of the drug in the fluid channel (115) results in the counter-electrode layer (108) being bent, thus modifying the capacitance of the sensing capacitor.

11. Cannula assembly (40) according to Claim 10, **characterized by** the electrode layer (106), the counter-electrode layer (108) and the fluid channel (115a, 115b) being arranged such that the capacitance increases upon increasing fluidic pressure of the drug in the fluid channel (115a, 115b).

12. Cannula assembly (40) according to Claim 10, **characterized by** the electrode layer (106), the counter-electrode layer (108) and the fluid channel (115a, 115b) being arranged such that the capacitance of the sensing capacitor decreases upon increasing fluidic pressure of the drug in the fluid channel (115a, 115b).

13. Cannula assembly according to either of the previous claims, **characterized by** the pressure sensor (15) comprising a further sensing capacitor, the further sensing capacitor comprising a further electrode layer and a further counter-electrode layer, the further electrode layer, the further counter-electrode layer and the fluid channel being arranged such that the pressure in the fluid channel modifies the capacitance of the sensing capacitor and the capacitance of the further sensing capacitor in opposed ways.

14. Ambulatory infusion system for the infusion of a liquid drug into a patient's body over an extended time period, the ambulatory infusion system comprising:
a) a drug container,
b) an infusion cannula (20) which is fluidically coupled to the drug container and is configured to be placed into the patient's subcutaneous tissue for drug infusion,
c) an infusion device (5), the infusion device (5) being coupled to the drug container for controlled drug administration via the infusion cannula,
d) a pressure sensor (15) with a fluid channel (115), the fluid channel being fluidically arranged between the drug container and the infusion cannula (20), the pressure sensor (15) defining a sensing capacitor,
e) a measuring unit (25) which is coupled to the pressure sensor (15), the measuring unit (25) being configured to evaluate a variation of the capacitance of the sensing capacitor,
**characterized by** the pressure sensor (15) comprises a stack (100) of coplanar layers, the stack of coplanar layers comprising:
f) a rigid top layer (110) and a rigid base layer (102),
g) a resilient metallic electrode layer (106) and a metallic counter-electrode layer (108), the electrode layer (106) and the counter-electrode layer (108) being electrodes of the sensing capacitor,
h) a spacer layer (109) which has a through cut-out, the through cut-out defining an electrode cavity (112, 112a),
wherein the fluid channel (115) is coupled to the electrode layer (106) such that a positive fluidic pressure of the drug in the fluid channel (115) causes the electrode layer (106) to bend into the electrode cavity (112, 112a), thus modifying the capacitance of the sensing capacitor, and
**wherein** the electrode layer (106) and the spacer layer (109) are made from foils and the layers which are arranged between the top layer (110) and the bottom layer (102) are of substantially the same size.

## Patentansprüche

1. Kanülenanordnung (40), umfassend eine Infusionskanüle (20) und eine Kanülenhub (50), wobei der Hub (50) einen Drucksensor (15) mit einem Fluidkanal (115) umfasst und der Fluidkanal (115) strömungstechnisch der Infusionskanüle (20) vorgelagert ist,
**dadurch gekennzeichnet, dass** der Drucksensor (15) einen Stapel (100) von koplanaren Schichten umfasst, wobei der Stapel koplanarer Schichten Folgendes umfasst:
a) eine starre Deckschicht (110) und eine starre Grundschicht (102),
b) eine elastische, metallische Elektrodenschicht (106) und eine metallische Gegenelektrodenschicht (108), wobei die Elektrodenschicht (106) und die Gegenelektrodenschicht (108) Elektroden eines Sensorkondensators sind,
c) eine Abstandshalterschicht (109), die einen durchgehenden Ausschnitt aufweist, wobei der durchgehende Ausschnitt eine Elektrodenkavität (112) definiert,
wobei der Fluidkanal (115) an die Elektrodenschicht (106) so gekoppelt ist, dass ein positiver, strömungstechnischer Druck des Arzneimittels im Fluidkanal (115) verursacht, dass die Elektrodenschicht (106) sich in die Elektrodenkavität (112) biegt und auf diese Weise die Kapazität des Sensorkondensators modifiziert, und
wobei die Elektrodenschicht (106) und die Abstandshalterschicht (109) aus Folien hergestellt sind, und die Schichten, die zwischen der Deckschicht (110) und der Grundschicht (102) angeordnet sind, von im Wesentlichen gleicher Größe sind.

2. Kanülenanordnung (40) nach Anspruch 1, **gekennzeichnet durch** einen strömungstechnischen Koppler, der dem Drucksensor (15) vorgelagert ist und in strömungstechnischer Kommunikation mit dem Fluidkanal (115) steht, wobei der strömungstechnische Koppler ausgelegt ist, um die Kanülenanordnung an eine Infusionsvorrichtung zu koppeln.

3. Kanülenanordnung (40) nach einem der vorstehenden Ansprüche, **dadurch gekennzeichnet, dass** die Abstandshalterschicht (109) zwischen und in Kontakt mit der Elektrodenschicht (106) und der Gegenelektrodenschicht (108) angeordnet ist.

4. Kanülenanordnung (40) nach Anspruch 3, **dadurch gekennzeichnet, dass** die Elektrodenschicht (106), die Gegenelektrodenschicht (108) und die Elektrodenkavität (112) so konstruiert sind, dass bei einem maximalen Schwellendruck die Elektrodenschicht (106) die Gegenelektrodenschicht (108) berührt.

5. Kanülenanordnung (40) nach Anspruch 4, **dadurch gekennzeichnet, dass** die Elektrodenschicht (106) und die Gegenelektrodenschicht (108) einen galvanischen Kontakt herstellen, wenn die Elektrodenschicht (106) die Gegenelektrodenschicht (108) berührt.

6. Kanülenanordnung (40) nach Anspruch 4, **dadurch gekennzeichnet, dass** mindestens eine von Elektrodenschicht (106) und Gegenelektrodenschicht (108) eine Isolierschicht (132) umfasst, die der anderen von Elektrodenschicht (106) und Gegenelektrodenschicht (108) jeweils gegenüberliegt und auf diese Weise verhindert, dass ein galvanischer Kontakt zwischen der Elektrodenschicht (106) und der Gegenelektrodenschicht (108) hergestellt wird.

7. Kanülenanordnung (40) nach einem der vorstehenden Ansprüche, **gekennzeichnet durch** einen Strömungsbegrenzer (17, 162) im Fluidkanal (115).

8. Kanülenanordnung (40) nach einem der vorstehenden Ansprüche, **gekennzeichnet durch** einen Referenzkondensator, der eine Referenzelektrodenschicht (106) und eine Referenzgegenelektrodenschicht (108') umfasst.

9. Kanülenanordnung (40) nach Anspruch 8, **dadurch gekennzeichnet, dass** eine von Elektrodenschicht (106) und Referenzelektrodenschicht (106) oder Gegenelektrodenschicht (108) und Referenzgegenelektrodenschicht (108') eine gemeinsame Schicht ist.

10. Kanülenanordnung (40) nach einem der vorstehenden Ansprüche, **dadurch gekennzeichnet, dass** der Fluidkanal (115a, 115b) an die Gegenelektrodenschicht (108) so gekoppelt ist, dass ein strömungstechnischer Druck des Arzneimittels im Fluidkanal (115) dazu führt, dass die Gegenelektrodenschicht (108) gebogen wird und auf diese Weise die Kapazität des Sensorkondensators modifiziert.

11. Kanülenanordnung (40) nach Anspruch 10, **dadurch gekennzeichnet, dass** die Elektrodenschicht (106), die Gegenelektrodenschicht (108) und der Fluidkanal (115a, 115b) so angeordnet sind, dass sich die Kapazität bei Erhöhen des strömungstechnischen Drucks des Arzneimittels im Fluidkanal (115a, 115b) erhöht.

12. Kanülenanordnung (40) nach Anspruch 10, **dadurch gekennzeichnet, dass** die Elektrodenschicht (106), die Gegenelektrodenschicht (108) und der Fluidkanal (115a, 115b) so angeordnet sind, dass die Kapazität des Sensorkondensators bei Erhöhen des strömungstechnischen Drucks des Arzneimittels im Fluidkanal (115a, 115b) abnimmt.

13. Kanülenanordnung nach einem der vorstehenden Ansprüche, **dadurch gekennzeichnet, dass** der Drucksensor (15) einen weiteren Sensorkondensator umfasst, wobei der weitere Sensorkondensator eine weitere Elektrodenschicht und eine weitere Gegenelektrodenschicht umfasst, die weitere Elektrodenschicht, die weitere Gegenelektrodenschicht und der Fluidkanal so angeordnet sind, dass der Druck im Fluidkanal die Kapazität des Sensorkondensators und die Kapazität des weiteren Sensorkondensators auf entgegengesetzte Weise modifiziert.

14. Ambulantes Infusionssystem zur Infusion eines flüssigen Arzneimittels in den Körper eines Patienten über einen längeren Zeitraum, wobei das ambulante Infusionssystem Folgendes umfasst:
a) einen Arzneimittelbehälter,
b) eine Infusionskanüle (20), die strömungstechnisch an den Arzneimittelbehälter gekoppelt und ausgelegt ist, um in das subkutane Gewebe des Patienten zur Arzneimittelinfusion platziert zu werden,
c) eine Infusionsvorrichtung (5), wobei die Infusionsvorrichtung (5) an den Arzneimittelbehälter zur kontrollierten Arzneimittelverabreichung über die Infusionskanüle gekoppelt wird,
d) einen Drucksensor (15) mit einem Fluidkanal (115), wobei der Fluidkanal strömungstechnisch zwischen dem Arzneimittelbehälter und der Infusionskanüle (20) angeordnet ist und der Drucksensor (15) einen Sensorkondensator definiert,
e) eine Messeinheit (25), die an den Drucksensor (15) gekoppelt ist, wobei die Messeinheit (25) ausgelegt ist, um eine Variation der Kapazität des Sensorkondensators zu bewerten,
**dadurch gekennzeichnet, dass** der Drucksensor (15) einen Stapel (100) von koplanaren Schichten umfasst, wobei der Stapel koplanarer Schichten Folgendes umfasst:
f) eine starre Deckschicht (110) und eine starre Grundschicht (102),
g) eine elastische, metallische Elektrodenschicht (106) und eine metallische Gegenelektrodenschicht (108), wobei die Elektrodenschicht (106) und die Gegenelektrodenschicht (108) Elektroden des Sensorkondensators sind,
h) eine Abstandshalterschicht (109), die einen durchgehenden Ausschnitt aufweist, wobei der durchgehende Ausschnitt eine Elektrodenkavität (112, 112a) definiert,
wobei der Fluidkanal (115) an die Elektrodenschicht (106) so gekoppelt ist, dass ein positiver, strömungstechnischer Druck des Arzneimittels im Fluidkanal (115) verursacht, dass die Elektrodenschicht (106) sich in die Elektrodenkavität (112, 112a) biegt und auf diese Weise die Kapazität des Sensorkondensators modifiziert, und
wobei die Elektrodenschicht (106) und die Abstandshalterschicht (109) aus Folien hergestellt sind, und die Schichten, die zwischen der Deckschicht (110) und der Bodenschicht (102) angeordnet sind, von im Wesentlichen gleicher Größe sind.

## Revendications

1. Ensemble canule (40), comprenant une canule de perfusion (20) et un raccord (50), le raccord (50) comprenant un capteur de pression (15) avec un canal de fluide (115), le canal de fluide (115) étant fluidiquement agencé en amont de la canule de perfusion (20),
**caractérisé en ce que** le capteur de pression (15) comprend un empilement (100) de couches coplanaires, l'empilement de couches coplanaires comprenant :
a) une couche supérieure rigide (110) et une couche de base rigide (102),
b) une couche d'électrode métallique résiliente (106) et une couche de contre-électrode métallique (108), la couche d'électrode (106) et la couche de contre électrode (108) étant des électrodes d'un condensateur de détection,
c) une couche d'espacement (109) qui a une découpe traversante, la découpe traversante définissant une cavité d'électrode (112),
dans lequel le canal de fluide (115) est couplé à la couche d'électrode (106) de telle sorte qu'une pression fluidique positive du médicament dans le canal de fluide (115) entraîne la flexion de la couche d'électrode (106) à l'intérieur de la cavité d'électrode (112), modifiant ainsi la capacité du condensateur de détection, et dans lequel la couche d'électrode (106) et la couche d'espacement (109) sont formées par des feuilles d'aluminium et les couches qui sont disposées entre la couche supérieure (110) et la couche inférieure (102) sont sensiblement de la même taille.

2. Ensemble canule (40) selon la revendication 1, **caractérisé par** un coupleur fluidique en amont du capteur de pression (15) et en communication fluidique avec le canal de fluide (115), le coupleur fluidique étant configuré pour coupler l'ensemble canule à un dispositif de perfusion.

3. Ensemble canule (40) selon l'une ou l'autre des revendications précédentes, **caractérisé en ce que** la couche d'espacement (109) est agencée entre et en contact avec la couche d'électrode (106) et la couche de contre-électrode (108).

4. Ensemble canule (40) selon la revendication 3, **caractérisé en ce que** la couche d'électrode (106), la couche de contre-électrode (108) et la cavité d'électrode sont conçues de telle sorte que, sous l'effet d'une pression seuil maximale, la couche d'électrode (106) touche la couche de contre-électrode (108) .

5. Ensemble canule (40) selon la revendication 4, **caractérisé en ce que** la couche d'électrode (106) et la couche de contre-électrode (108) établissent un contact galvanique lorsque la couche d'électrode (106) touche la couche de contre-électrode (108).

6. Ensemble canule (40) selon la revendication 4, **caractérisé en ce qu'**au moins l'une de la couche d'électrode (106) et la couche de contre-électrode (108) comprend une couche d'isolation (132) face à l'autre de la couche d'électrode (106) et de la couche de contre-électrode (108), respectivement, empêchant ainsi l'établissement d'un contact galvanique par la couche d'électrode (106) et la couche de contre-électrode (108) .

7. Ensemble canule (40) selon l'une quelconque des revendications précédentes, **caractérisé par** un restricteur de flux (17, 162) dans le canal de fluide (115) .

8. Ensemble canule (40) selon l'une quelconque des revendications précédentes, **caractérisé par** un condensateur de référence comprenant une couche d'électrode de référence (106) et une couche de contre-électrode de référence (108').

9. Ensemble canule (40) selon la revendication 8, **caractérisé par** l'une ou l'autre de la couche d'électrode (106) et de la couche d'électrode de référence (106) ou la couche de contre-électrode (108) et la couche de contre-électrode de référence (108') étant une couche commune.

10. Ensemble canule (40) selon l'une quelconque des revendications précédentes, **caractérisé en ce que** le canal de fluide (115a, 115b) est couplé à la couche de contre-électrode (108) de telle sorte qu'une pression fluidique du médicament dans le canal de fluide (115) résulte en la flexion de la couche de contre-électrode (108), modifiant ainsi la capacité du condensateur de détection.

11. Ensemble canule (40) selon la revendication 10, **caractérisé en ce que** la couche d'électrode (106), la couche de contre-électrode (108) et le canal de fluide (115a, 115b) sont agencés de telle sorte que la capacité augmente lors de l'augmentation de la pression fluidique du médicament dans le canal de fluide (115a, 115b) .

12. Ensemble canule (40) selon la revendication 10, **caractérisé en ce que** la couche d'électrode (106), la couche de contre-électrode (108) et le canal de fluide (115a, 115b) sont agencés de telle sorte que la capacité du condensateur de détection diminue lors de l'augmentation de la pression fluidique du médicament dans le canal de fluide (115a, 115b).

13. Ensemble canule selon l'une quelconque des revendications précédentes, **caractérisé en ce que** le capteur de pression (15) comprend un condensateur de détection supplémentaire, le condensateur de détection supplémentaire comprenant une couche d'électrode supplémentaire et une couche de contre-électrode supplémentaire, la couche d'électrode supplémentaire, la couche de contre-électrode supplémentaire et le canal de fluide étant agencés de telle sorte que la pression dans le canal de fluide modifie la capacité du condensateur de détection et la capacité du condensateur de détection supplémentaire de manière opposée.

14. Système de perfusion ambulatoire pour la perfusion d'un médicament liquide dans le corps d'un patient sur une période de temps prolongée, le système de perfusion ambulatoire comprenant :
a) un récipient de médicament,
b) une canule de perfusion (20) qui est fluidiquement couplée au récipient de médicament et est configurée pour être placée dans le tissu sous-cutané du patient pour la perfusion de médicaments,
c) un dispositif de perfusion (5), le dispositif de perfusion (5) étant couplé au récipient de médicament pour l'administration de médicament régulée via la canule de perfusion,
d) un capteur de pression (15) avec un canal de fluide (115), le canal de fluide étant fluidiquement agencé entre le récipient de médicament et la canule de perfusion (20), le capteur de pression (15) définissant un condensateur de détection,
e) une unité de mesure (25) qui est couplée au capteur de pression (15), l'unité de mesure (25) étant configurée pour évaluer une variation de la capacité du condensateur de détection,
**caractérisé en ce que** le capteur de pression (15) comprend un empilement (100) de couches coplanaires, l'empilement de couches coplanaires comprenant :
f) une couche supérieure rigide (110) et une couche de base rigide (102),
g) une couche d'électrode métallique résiliente (106) et une couche de contre-électrode métallique (108), la couche d'électrode (106) et la couche de contre électrode (108) étant des électrodes du premier condensateur de détection,
h) une couche d'espacement (109) qui a une découpe traversante, la découpe traversante définissant une cavité d'électrode (112, 112a),
dans lequel le canal de fluide (115) est couplé à la couche d'électrode (106) de telle sorte qu'une pression fluidique positive du médicament dans le canal de fluide (115) entraîne la flexion de la couche d'électrode (106) à l'intérieur de la cavité d'électrode (112, 112a), modifiant ainsi la capacité du condensateur de détection, et
dans lequel la couche d'électrode (106) et la couche d'espacement (109) sont formées par des feuilles d'aluminium et les couches qui sont disposées entre la couche supérieure (110) et la couche inférieure (102) sont sensiblement de la même taille.
